(19) Europäisches Patentamt
European Patent Office
Office européen des brevets

(11) EP 4 480 942 B1

(12) **EUROPEAN PATENT SPECIFICATION**

(45) Date of publication and mention of the grant of the patent:
**08.07.2026 Bulletin 2026/28**

(21) Application number: **22926674.7**

(22) Date of filing: **08.07.2022**

(51) International Patent Classification (IPC):
***C07C 67/62*** *(2006.01)* ***C07C 69/82*** *(2006.01)*
***C08L 67/00*** *(2006.01)* ***C08J 11/18*** *(2006.01)*
***C08J 11/28*** *(2006.01)* ***C08J 11/24*** *(2006.01)*
***B01J 27/24*** *(2006.01)* ***C08B 16/00*** *(2006.01)*

(52) Cooperative Patent Classification (CPC):
(C-Sets available)
**C08J 11/28; B01J 31/0241; C07C 67/03;**
**C08J 11/24;** C08J 2367/00; Y02W 30/62 (Cont.)

(86) International application number:
**PCT/CN2022/104576**

(87) International publication number:
**WO 2023/155367 (24.08.2023 Gazette 2023/34)**

(54) **METHOD FOR RECYCLING BLENDED FABRIC CONTAINING POLYESTER**

VERFAHREN ZUR WIEDERVERWERTUNG VON POLYESTERHALTIGEM MISCHGEWEBE

PROCÉDÉ DE RECYCLAGE DE TISSU MÉLANGÉ CONTENANT DU POLYESTER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2022 CN 202210150394**

(43) Date of publication of application:
**25.12.2024 Bulletin 2024/52**

(73) Proprietor: **Qingdao Amino Material Technology Co. Ltd**
**Qingdao, Shandong 266399 (CN)**

(72) Inventors:
- **LIU, Honggang**
  **Qingdao, Shandong 266399 (CN)**
- **GUO, Qing**
  **Qingdao, Shandong 266399 (CN)**
- **GUO, Caihong**
  **Qingdao, Shandong 266399 (CN)**
- **MAO, Debin**
  **Qingdao, Shandong 266399 (CN)**
- **HU, Wenjian**
  **Qingdao, Shandong 266399 (CN)**
- **WAN, Xiaobo**
  **Qingdao, Shandong 266399 (CN)**
- **MU, Youbing**
  **Qingdao, Shandong 266399 (CN)**

(74) Representative: **Bals & Vogel Patentanwälte PartGmbB**
**Konrad-Zuse-Str. 4**
**44801 Bochum (DE)**

(56) References cited:
**WO-A1-2019/140245**
**WO-A1-2020/035590**
**WO-A1-2021/126661**
**WO-A1-2021/126661**
**CN-A- 102 250 379**
**CN-A- 103 930 395**
**CN-A- 105 658 611**
**CN-A- 108 602 974**
**US-A1- 2011 004 014**
**US-B2- 9 255 194**

- **ANNA PALME ET AL: "Development of an efficient route for combined recycling of PET and cotton from mixed fabrics", TEXTILES AND CLOTHING SUSTAINABILITY, 1 January 2017 (2017-01-01), pages 1 - 9, XP055675406, Retrieved from the Internet <URL:xx> [retrieved on 20200310], DOI: 10.1186/s40689-017-0026-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 67/03, C07C 69/82**

## Description

### TECHNICAL FIELD

The present invention relates to the recycling of fabrics, in particular to a method for recycling a polyester blend fabric.

### BACKGROUND

Polyester is one of the most popular fiber materials in the world. Since 2010, polyester fiber has been the most consumed fiber globally. In 2020, global annual polyester production reached 57 million tons. As a textile fiber, the polyester is generally blended with other fibers in addition to being used alone. A blended polyester fabric not only shows the excellent mechanical properties of polyester fabrics, but also obviously retains the excellent properties of other fiber fabrics. Therefore, various blend fabrics of polyester are becoming increasingly popular in the apparel industry. For example, the polyester-cotton blend fabric is one of the most common fabrics used in casual clothing. This blend fabric has the comfort of cotton as well as the cheapness of polyester fabrics. In sportswear, polyester-polyurethane blend fabrics dominate. Such blend fabrics retain the appearance and texture of polyester fiber while having excellent elasticity and wearing comfort. However, a large amount of clothing made from blend fabrics is discarded after use. This is due to the lack of technology in the prior art that could completely separate the polyester from other fiber components. As a result, millions of tons of polyester blend fabrics are simply incinerated and landfilled annually. Such a treatment process not only wastes a lot of resources but also causes a serious negative effect on our living environment. If polyester blended textile waste could be recycled into high value-added textile fibers through sustainable processes, not only the negative effect on the environment caused by waste disposal could be reduced, but also the limited resources could be used maximally.

Among the currently disclosed recycling technologies for polyester blend fabrics, chemical depolymerization of polyester is the most important process. However, the depolymerization temperature is generally as high as 200 °C or more during the depolymerization of polyester fibers (such as CN106113319A and CN102250379B). At this temperature, temperature-sensitive components are degraded earlier than the polyester, making it difficult to recycle other components in the blend fabric. Patent US8541477B2 disclosed a method for depolymerizing polyester at low temperature. In this method, the polyester could be depolymerized at 120 °C or more. However, temperature-sensitive components such as polyurethane begin to become sticky at 120 °C, making resulting separated polyurethane components lose their elasticity and become difficult to be recycled. WO2021126661A1 disclosed a method for depolymerizing polyester at a temperature of 100 °C to 180 °C. However, in the method, 100% depolymerization of the polyester could not be achieved at low temperature (100 °C to 140 °C). This prevents the polyester from being completely separated out of the blend fabric, making it difficult to recycle other fabric components. Moreover, this method results in a low yield for monomer from polyester at low temperature, which also makes the economic cost of the recycling higher. In view of this, regarding realizing the recycling of polyester blend fabrics, it is key to achieve 100% depolymerization of polyester components at low temperature without damaging other components.

Palme, A., et al., in "Development of an efficient route for combined recycling pf PET and cotton from mixed fabrics", Text Cloth Sustain 3, 4 (2017) discloses the separation of PET and cotton from mixed textiles.

WO2019140245A1 discloses systems and methods that involve a subcritical water reaction to recycle the cellulose and polyester components of waste cotton and cotton/polyester blend textiles that would otherwise be discarded or disposed of. The methods provide for treatment of the waste textiles to produce advanced materials including cellulose and terephthalic acid (TPA) with a low environmental impact.

WO2020035590A1 discloses a method for recycling polyester from a polyester textile. The method comprises the steps of: providing said polyester textile soaked in a mixture comprising a solvent and a catalyst, providing and maintaining a temperature of said mixture comprising said polyester textile within a range of 80-240 °C during depolymerization of polyester in said polyester textile and wherein, in said step of providing said polyester textile soaked in said mixture, said catalyst of said mixture comprises calcium hydroxide.

US 9 255 194 B2 discloses a method of depolymerizing a polyester by glycolysis in the presence of an amine organocatalyst such as 1,4-Diazabicyclo (2,2,2)octane, wherein the polyester can be in the form of a fiber or fabric and can contain other fibers such as cotton.

WO2021/126661 A1 discloses a process for the depolymerization of a poly($C_2$-$C_4$ alkylene terephthalate), which comprises contacting a poly($C_2$-$C_4$ alkylene terephthalate) with methanol and 1,8-diazabicyclo[5.4.0]undec-7-ene or triazabicyclodecene as catalyst at a temperature, which ranges between 100°C to 180°C.

### SUMMARY

An object of the present invention is to provide a method for recycling a polyester blend fabric. Specifically, the

present invention is according to claim 1. Specific embodiments of the invention are according to dependent claims 2 to 4.

To achieve the above object, the present invention adopts the following technical solutions:

The present invention provides a method for recycling a polyester blend fabric, including the following steps: treating a polyester-containing raw material to be treated, subjecting a resulting treated polyester-containing raw material to polyester degradation at a temperature of 40 °C to 120 °C for 0.5 h to 8 h in the presence of a catalyst, to achieve complete depolymerization, separating out and recovering a polyester component, and reutilizing the polyester component and other blended components.

In the present invention, the catalyst consists of an organic alkali main catalyst and an auxiliary catalyst, and the catalyst is in an amount of 0.1 wt% to 20 wt% of a mass of the polyester-containing raw material to be treated; and a mass ratio of the organic alkali main catalyst to the auxiliary catalyst ranges from 1 : 0.01 to 1 : 100.

In the present invention, the organic alkali main catalyst is at least one selected from the group consisting of a nitrogen-containing amidine compound, a nitrogen-containing guanidine compound, and derivatives thereof; and the auxiliary catalyst is a nitrile compound.

In some embodiments of the present invention, the organic alkali main catalyst is selected from the group consisting of: a) 1,8-diazabicyclo[5.4.0]undec-7-ene (DBU), a DBU-supported polymer or compound, and an organic salt formed from DBU and imidazole, and an organic salt formed from DBU and a derivative of imidazole; b) 1,5,7-triazabicyclo [4.4.0]dec-5-ene (TBD), a TBD-supported polymer or compound, and an organic salt formed from TBD and imidazole, and an organic salt formed from TBD and a derivative of imidazole; and c) 1,5-diazabicyclo[4.3.0]non-5-ene (DBN), a DBN-supported polymer or compound, an organic salt formed from DBN and imidazole, and an organic salt formed from DBN and a derivative of imidazole.

In some embodiments, the polymer-supporting or compound-supporting catalyst (taking the DBU-supported polymer or compound as an example) is DBU-supported polystyrene (PS-DBU), DBU-supported activated carbon.

The organic salt (taking the organic salt formed from DBU and imidazole, and the organic salt formed from DBU and a derivative of imidazole as an example) is as follows: an organic salt formed from DBU and imidazole ([HDBU][Im]), an organic salt formed from DBU and 2-ethylimidazole ([HDBU][2-Etlm]), an organic salt formed from DBU and benzimidazole ([HDBU][Blm]), an organic salt formed from DBU and 2-methylimidazole ([HDBU][2-Melm]), an organic salt formed from DBU and 2-ethyl-4-methylimidazole ([HDBU][2-Et-4-Melm]), and an organic salt formed from DBU and 2-phenylimidazole ([HDBU][2-Phlm]).

In some embodiments of the present invention, the auxiliary catalyst is at least one selected from the group consisting of acetonitrile, propionitrile, benzonitrile, and adiponitrile.

In the invention, in the method, the polyester-containing raw material after being treated is added into a methanol solution, and a resulting mixture is subjected to polyester degradation at a temperature of 60 °C to 110 °C for 1 h to 5 h in the presence of the catalyst, to achieve complete depolymerization; and the polyester component is separated out, recovered, and reutilized.

In some embodiments, the polyester-containing raw material after being treated is added into a methanol solution, and a resulting mixture is subjected to polyester degradation at a temperature of 80 °C to 110 °C for 1 h to 2 h in the presence of a catalyst to achieve complete depolymerization; and the polyester component is separated out, recovered, and reutilized.

In some embodiments of the present invention, the polyester-containing raw material to be treated is a mixed fabric of polyester fabric with other fabrics; the mixed fabric includes 5% to 100% of a polyester; and the polyester blend fabric is a fabric prepared by blend-spinning the polyester with at least one selected from the group consisting of polyurethane, cotton, viscose fiber, regenerated cellulose fiber, nylon, wool, cashmere, and silk.

In some embodiments, the polyester blend fabric as described above has a polyester content of at least 5%, or at least 25%, or at least 50%, or at least 75%, or at least 95%.

In the present invention, the polyester-containing raw material to be treated is washed with an alcohol or an alcohol-containing mixed system at a temperature of 40 °C to 130 °C for 0.3 h to 2 h; the alcohol-containing mixed system is a mixture of an alcohol with an alkaline compound, and the alkaline compound is at least one selected from the group consisting of sodium carbonate ($Na_2CO_3$), sodium acetate, sodium methoxide, sodium ethoxide, potassium carbonate, potassium acetate, potassium methoxide, calcium oxide, and calcium hydroxide.

In some embodiments, the raw material is washed with the alcohol or the alcohol-containing mixed system at a temperature of 50 °C to 110 °C, or 80 °C to 100 °C, or 90 °C to 100 °C for 0.3 h to 2 h, thereby removing stains, oil stains, and other impurities attached to the raw material.

In the present invention, solid-liquid separation is conducted after the polyester degradation, and a resulting solid is collected and subjected to Soxhlet extraction to separate the polyester component from the other fiber components; a resulting liquid is subjected to atmospheric distillation to recover an alcohol and the auxiliary catalyst, and a resulting residue after the atmospheric distillation is then subjected to vacuum distillation or extraction to recover a diol, and a binary acid, monomers from polyester, as well as the organic alkali main catalyst. In some embodiments, the monomers recovered after the polyester depolymerization, such as dimethyl terephthalate (DMT) and ethylene glycol, could be

purified by vacuum distillation and used as raw materials to synthesize a new polyester.

The embodiments as described above could be combined randomly.

Function mechanism of the present invention:

In the process of alcoholyzing the polyester with methanol, which is catalyzed by the organic alkali main catalyst, the organic alkali main catalyst first activates the methanol, such that the methanol generates methanol anions with strong nucleophilicity, as shown in the scheme below:

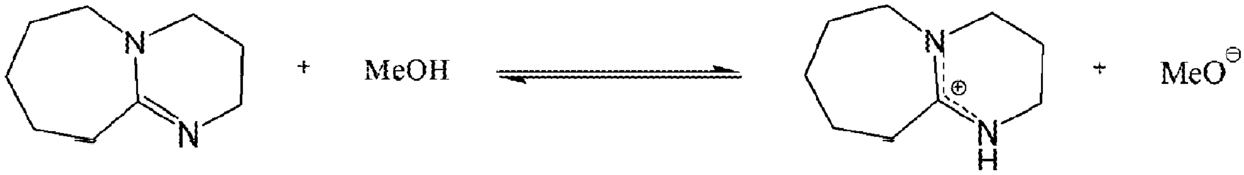

The formed methanol anions are more likely to attack an ester bond through nucleophilic reaction, thereby achieving cleavage of the ester bond. However, the reaction of activating methanol by the organic alkali main catalyst is reversible. When nitrile (R-C=N:) is added to the reaction system, a strong polarity of -C=N: and lone pair electrons on the nitrogen atom could stabilize organic alkali main catalyst cations generated from the reaction in the system. Therefore, the reaction equilibrium shifts toward the direction of producing more methanol anions, thereby improving the catalytic activity of the organic alkali main catalyst.

Some embodiments of the present invention have the following advantages:

In the present invention, the method achieves 100% depolymerization of polyester at low temperature. A resulting polyester component after depolymerization could be easily separated from other components, thereby realizing complete separation of the polyester component from other temperature-sensitive fiber components in a blend fabric. Moreover, the separation is efficient and could be performed under gentle conditions; other fibers could be obtained with 100% pure components and are almost not damaged, and could be directly re-spun into yarns or used as raw materials to produce regenerated fibers. Monomers recovered from polyester depolymerization could also be used for the synthesis of a new polyester, thus achieving the closed-loop recycling of different fiber components in a polyester blended waste. Therefore, the recycling method is of great significance to protecting the ecological environment and reducing the cost of recycled fibers.

In the present invention, an organic alkaline catalyst is used in the separation to catalyze the cleavage of polyester ester bonds. In order to improve the catalytic activity and efficiency of organic alkali at low temperature, the auxiliary catalyst is added during the system catalysis. The organic alkali, in conjunction with the auxiliary catalyst, achieves 100% depolymerization of polyester at low temperature. Moreover, a monomer recovery rate of polyester is significantly improved after adding the auxiliary catalyst.

## BRIEF DESCRIPTION OF THE DRAWINGS

FIG. 1 shows a Fourier transform infrared (FTIR) spectroscopy spectrum of the monomer (DMT) from polyester recovered after depolymerization provided in an example of the present disclosure.

FIG. 2 shows an FTIR spectroscopy spectrum of the monomer dimethyl furan-2,5-dicarboxylate from polyester recovered after depolymerization provided in an example of the present disclosure.

FIG. 3 shows the separation and recovery of a polyester-cotton blend fabric provided in an example of the present disclosure, in which, A shows the blend fabric before reaction and separation; and B shows a pure cotton fabric after reaction and separation.

FIG. 4 shows the separation and recovery of a polyester-polyurethane blend printed fabric provided in an example of the present disclosure, in which A shows the blend fabric before reaction and separation; B shows a pure polyurethane fabric after reaction and separation; and C shows that polyurethane filaments are not damaged under an optical microscope.

## DETAILED DESCRIPTION OF THE EMBODIMENTS

Specific embodiments of the present invention will be further described below in conjunction with examples.

In the present invention, unless otherwise specified, the term "complete depolymerization" refers to 100% depolymerization of polyester.

In the present invention, the method achieves 100% depolymerization of polyester fiber components at a lower

temperature, and the polyester monomer has a recovery rate as high as 95%, thereby enabling more efficient recovery and regeneration of polyester. Lower reaction temperatures make the process more environmentally-friendly and reduce the energy required for the reaction, thus making the recycling have lower cost.

The present invention aims to recycle waste materials made by blend-spinning temperature-sensitive fibers with polyester, for example, polyurethane-polyester blend fabrics, cotton-polyester blend fabrics, viscose fiber-polyester blend fabrics, regenerated cellulose fiber-polyester blend fabrics, nylon-polyester blend fabrics, wool-polyester blend fabrics, cashmere-polyester blend fabrics, silk-polyester blend fabrics, and mixed fabrics of polyester fabric and fabrics of these fibers. The regenerated cellulose fiber here may be Modal fiber and Lyocell fiber.

The polyester depolymerization refers to the degradation of polyester into monomers and small-molecule oligomers, such as dimers, trimers, tetramers, pentamers, hexamers, and heptamers, thereby falling off and separating from blend fabrics, so as to achieve separation and recovery of polyester component(s). The polyester is a polymer containing ester chemical bonds, such as poly(ethylene terephthalate) (PET), poly(trimethylene terephthalate) (PTT), poly(butylene terephthalate) (PBT), poly(ethylene-2,5-furandicarboxylate) (PEF), poly(trimethylene 2,5-furandicarboxylate) (PTF), poly(butylene 2,5-furandicarboxylate) (PBF), polylactate (PLA), polyhydroxyalkanoate (PHA), poly(butylene succinate) (PBS), and low-melting-point polyester copolymer.

Depolymerization efficiency (E) can be calculated from the amount of solids remaining after the reaction. A calculation equation is as follows:

Depolymerization efficiency (E) = (polyester fiber content in textile fabric - amount of undepolymerized polyester)/ polyester fiber content in textile fabric.

The preparation of an organic salt catalyst in the following examples is as follows:

Preparation of an organic salt catalyst formed from DBU with imidazole and a derivative thereof:

The organic salt catalyst formed from the DBU with imidazole and a derivative thereof is prepared through neutralization reaction of equimolarDBU with imidazole and a derivative thereof at room temperature. For example, the organic salt ([HDBU][2-EtIm]) formed from DBU and 2-ethylimidazole used in the examples is prepared through neutralization reaction of 0.025 mol of DBU with 0.025 mol of 2-ethylimidazole at room temperature for 5 h.

Similarly, the following compounds can be prepared according to the above method: organic salt formed from DBU with benzimidazole ([HDBU][BIm]), catalyst formed from DBU with 2-methylimidazole ([HDBU][2-MeIm]), catalyst formed from DBU with 2-ethyl-4-methylimidazole ([HDBU][2-Et-4-MeIm]), and catalyst formed from DBU with 2-phenylimidazole ([HDBU][2-PhIm]).

**Reference Example 1**

10 g of polyester fibers were washed with methanol at 50 °C for 30 min. 0.2 g of sodium carbonate was added during the washing, and the washed polyester fibers were dried at 75 °C for 1 h. The dried polyester fibers, 20 g of methanol, 20 g of acetonitrile, and 2 g of DBU were added into a reaction kettle and heated to 65 °C. After 8 h of reaction, the reaction kettle was cooled to room temperature. The reaction system was filtered, to separate out a solid part and a liquid part. The solid part was subjected to Soxhlet extraction with methanol to obtain 8.94 g of DMT, i.e., a monomer from polyester depolymerization (shown in FIG. 1) without remaining solid matter in the Soxhlet extractor, indicating that the polyester fibers were completely depolymerized. The liquid part was subjected to distillation to recover the methanol and acetonitrile, and ethylene glycol and DBU were recovered through further vacuum distillation (shown in Table 2).

**Example 2** - **not according to the present invention-**

10 g of polyester fibers were washed with methanol at 50 °C for 30 min. 0.2 g of sodium carbonate was added during the washing, and the washed polyester fibers were dried at 75 °C for 1 h. The dried polyester fibers, 28 g of methanol, 12 g of different auxiliary catalysts, and 0.54 g of DBU were added into a reaction kettle and heated to 85 °C. After 2.5 h of reaction, the reaction kettle was cooled to room temperature. The reaction system was filtered, to separate out a solid part and a liquid part. The solid part was subjected to Soxhlet extraction with methanol to obtain DMT, i.e., a monomer from polyester depolymerization, and the remaining solid in the Soxhlet extractor was undepolymerized polyester fibers (shown in Table 1).

After depolymerization using acetonitrile as the auxiliary catalyst, the reaction system was filtered, to separate out a solid part and a liquid part. The solid part was subjected to Soxhlet extraction with methanol to obtain 9.35 g of DMT, i.e., a monomer from polyester depolymerization, without remaining solid matter in the Soxhlet extractor, indicating that the polyester fibers were completely depolymerized. The liquid part was subjected to distillation to recover the methanol and acetonitrile, and ethylene glycol and DBU were recovered through further vacuum distillation (shown in Table 2).

Table 1 Comparison among activities of different auxiliary catalysts for DBU catalytic degradation of polyester at low temperature

| Auxiliary catalyst | Reaction temperatu re °C | DBU wt% | m (Compoun d)/m (DBU) | m (Methanol )/m (Polyester ) | Reactio n time, h | Polyester depolymeriza tion rate, % | DMT recover y rate, mol% |
|---|---|---|---|---|---|---|---|
| None | 85 | 5.4 | 0 | 2.8 | 2.5 | 49.3 | 39.55 |
| Zinc acetate | 85 | 5.4 | 22.2 | 2.8 | 2.5 | 0 | 0 |
| 2-methylpyridine | 85 | 5.4 | 22.2 | 2.8 | 2.5 | 10.48 | 0 |
| Triethylamine | 85 | 5.4 | 22.2 | 2.8 | 2.5 | 40.85 | 31.62 |
| Dimethyl sulfoxide | 85 | 5.4 | 22.2 | 2.8 | 2.5 | 41.90 | 29.43 |
| 2-methylfuran | 85 | 5.4 | 22.2 | 2.8 | 2.5 | 60.41 | 48.68 |
| Tetrahydrofuran | 85 | 5.4 | 22.2 | 2.8 | 2.5 | 69.52 | 57.68 |
| N,N-Dimethylformam ide | 85 | 5.4 | 22.2 | 2.8 | 2.5 | 76.75 | 61.23 |
| Pyridine | 85 | 5.4 | 22.2 | 2.8 | 2.5 | 78.21 | 63.72 |
| Acetonitrile | 85 | 5.4 | 22.2 | 2.8 | 2.5 | 100 | 92.68 |

**Reference Example 3**

10 g of polyester fibers were washed with methanol at 50 °C for 30 min. 0.2 g of sodium carbonate was added during the washing, and the washed polyester fibers were dried at 75 °C for 1 h. The dried polyester fibers, 28 g of methanol, 12 g of benzonitrile, and 0.54 g of DBU were added into a reaction kettle and heated to 85 °C. After 2.5 h of reaction, the reaction kettle was cooled to room temperature. The reaction system was filtered, to separate out a solid part and a liquid part. The solid part was subjected to Soxhlet extraction with methanol to obtain 8.67 g of DMT, i.e., a monomer from polyester depolymerization, without remaining solid matter in the Soxhlet extractor, indicating that the polyester fibers were completely depolymerized. The liquid part was subjected to distillation to recover the methanol, and benzonitrile, ethylene glycol, and DBU were recovered through further vacuum distillation (shown in Table 2).

**Reference Example 4**

2 g of polyester fibers were washed with methanol at 50 °C for 30 min. 0.1 g of sodium carbonate was added during the washing, and the washed polyester fibers were dried at 75 °C for 1 h. The dried polyester fibers, 6.4 g of methanol, 1.6 g of acetonitrile, and 0.14 g of [HDBU][2-Etlm] were added into a reaction kettle and heated to 90 °C. After 2 h of reaction, the reaction kettle was cooled to room temperature. The reaction system was filtered, to separate out a solid part and a liquid part. The solid part was subjected to Soxhlet extraction with methanol to obtain 1.74 g of DMT, i.e., a monomer from polyester depolymerization, without remaining solid matter in the Soxhlet extractor, indicating that the polyester fibers were completely depolymerized. The liquid part was subjected to distillation to recover the methanol, acetonitrile, and ethylene glycol, and [HDBU][2-Etlm] was recovered through further extraction (shown in Table 2).

**Reference Example 5**

10 g of polyester fibers were washed with methanol at 50 °C for 30 min. 0.2 g of sodium carbonate was added during the washing, and the washed polyester fibers were dried at 75 °C for 1 h. The dried polyester fibers, 36 g of methanol, 4 g of acetonitrile, and 0.3 g of DBU were added into a reaction kettle and heated to 95 °C. After 2.5 h of reaction, the reaction kettle was cooled to room temperature. The reaction system was filtered, to separate out a solid part and a liquid part. The solid part was subjected to Soxhlet extraction with methanol to obtain 9.60 g of DMT, i.e., a monomer from polyester depolymerization, without remaining solid matter in the Soxhlet extractor, indicating that the polyester fibers were completely depolymerized. The liquid part was subjected to distillation to recover the methanol and acetonitrile, and ethylene glycol and DBU were recovered through further vacuum distillation (shown in Table 2).

**Reference Example 6**

10 g of polyester fibers were washed with methanol at 50 °C for 30 min. 0.2 g of sodium carbonate was added during the washing, and the washed polyester fibers were dried at 75 °C for 1 h. The dried polyester fibers, 36 g of methanol, 4 g of adiponitrile, and 0.3 g of DBU were added into a reaction kettle and heated to 95 °C. After 2.5 h of reaction, the reaction kettle was cooled to room temperature. The reaction system was filtered, to separate out a solid part and a liquid part. The solid part was subjected to Soxhlet extraction with methanol to obtain 8.55 g of DMT, i.e., a monomer from polyester depolymerization, without remaining solid matter in the Soxhlet extractor, indicating that the polyester fibers were completely depolymerized. The liquid part was subjected to distillation to recover the methanol, and ethylene glycol, adiponitrile, and DBU were recovered through further vacuum distillation (shown in Table 2).

**Reference Example 7**

10 g of polyester fibers were washed with methanol at 50 °C for 30 min. 0.2 g of sodium carbonate was added during the washing, and the washed polyester fibers were dried at 75 °C for 1 h. The dried polyester fibers, 28 g of methanol, 12 g of acetonitrile, and 0.18 g of DBU were added into a reaction kettle and heated to 115 °C. After 2 h of reaction, the reaction kettle was cooled to room temperature. The reaction system was filtered, to separate out a solid part and a liquid part. The solid part was subjected to Soxhlet extraction with methanol to obtain 8.61 g of DMT, i.e., a monomer from polyester depolymerization, without remaining solid matter in the Soxhlet extractor, indicating that the polyester fibers were completely depolymerized. The liquid part was subjected to distillation to recover the methanol and acetonitrile, and ethylene glycol and DBU were recovered through further vacuum distillation (shown in Table 2).

**Reference Example 8**

10 g of polyester fibers were washed with methanol at 50 °C for 30 min. 0.2 g of sodium carbonate was added during the washing, and the washed polyester fibers were dried at 75 °C for 1 h. The dried polyester fibers, 28 g of methanol, 12 g of propionitrile, and 0.18 g of DBU were added into a reaction kettle and heated to 115 °C. After 2 h of reaction, the reaction kettle was cooled to room temperature. The reaction system was filtered, to separate out a solid part and a liquid part. The solid part was subjected to Soxhlet extraction with methanol to obtain 7.79 g of DMT, i.e., a monomer from polyester depolymerization, without remaining solid matter in the Soxhlet extractor, indicating that the polyester fibers were completely depolymerized. The liquid part was subjected to distillation to recover the methanol and propionitrile, and ethylene glycol and DBU were recovered through further vacuum distillation (shown in Table 2).

**Reference Example 9**

10 g of polyester fibers were washed with methanol at 50 °C for 30 min. 0.2 g of sodium carbonate was added during the washing, and the washed polyester fibers were dried at 75 °C for 1 h. The dried polyester fibers, 28 g of methanol, 12 g of acetonitrile, and 0.18 g of TBD were added into a reaction kettle and heated to 115 °C. After 2 h of reaction, the reaction kettle was cooled to room temperature. The reaction system was filtered, to separate out a solid part and a liquid part. The solid part was subjected to Soxhlet extraction with methanol to obtain 8.36 g of DMT, i.e., a monomer from polyester depolymerization, without remaining solid matter in the Soxhlet extractor, indicating that the polyester fibers were completely depolymerized. The liquid part was subjected to distillation to recover the methanol and acetonitrile, and ethylene glycol and TBD were covered through further vacuum distillation (shown in Table 2).

**Comparative Example 1**

10 g of polyester fibers were washed with methanol at 50 °C for 30 min. 0.2 g of sodium carbonate was added during the washing, and the washed polyester fibers were dried at 75 °C for 1 h. The dried polyester fibers, 40 g of methanol and 2 g of DBU were added into a reaction kettle and heated to 65 °C. After 8 h of reaction, the reaction kettle was cooled to room temperature. The reaction system was filtered, to separate out a solid part and a liquid part. The solid part was subjected to Soxhlet extraction with methanol to obtain 2.81 g of DMT, i.e., a monomer from polyester depolymerization. 4.55 g of undepolymerized polyester fibers were remained in the Soxhlet extractor. The liquid part was subjected to distillation to recover the methanol, ethylene glycol, and DBU (shown in Table 2).

**Comparative Example 2**

10 g of polyester fibers were washed with methanol at 50 °C for 30 min. 0.2 g of sodium carbonate was added during the washing, and the washed polyester fibers were dried at 75 °C for 1 h. The dried polyester fibers, 40 g of methanol

and 0.54 g of DBU were added into a reaction kettle and heated to 85 °C. After 2.5 h of reaction, the reaction kettle was cooled to room temperature. The reaction system was filtered, to separate out a solid part and a liquid part. The solid part was subjected to Soxhlet extraction with methanol to obtain 3.99 g of DMT, i.e., a monomer from polyester depolymerization. 5.07 g of undepolymerized polyester fibers were remained in the Soxhlet extractor. The liquid part was subjected to distillation to recover the methanol, ethylene glycol, and DBU (shown in Table 2).

**Comparative Example 3**

2 g of polyester fibers were washed with methanol at 50 °C for 30 min. 0.1 g of sodium carbonate was added during the washing, and the washed polyester fibers were dried at 75 °C for 1 h. The dried polyester fibers, 8 g of methanol, and 0.14 g of [HDBU][2-Etlm] were added into a reaction kettle and heated to 90 °C. After 2 h of reaction, the reaction kettle was cooled to room temperature. The reaction system was filtered, to separate out a solid part and a liquid part. The solid part was subjected to Soxhlet extraction with methanol to obtain 0.91 g of DMT, i.e., a monomer from polyester depolymerization. 0.75 g of undepolymerized polyester fibers were remained in the Soxhlet extractor. The liquid part was subjected to distillation to recover the methanol and ethylene glycol, and [HDBU][2-Etlm] was recovered through further extraction (shown in Table 2).

**Comparative Example 4**

10 g of polyester fibers were washed with methanol at 50 °C for 30 min. 0.2 g of sodium carbonate was added during the washing, and the washed polyester fibers were dried at 75 °C for 1 h. The dried polyester fibers, 40 g of methanol, and 0.3 g of DBU were added into a reaction kettle and heated to 95 °C. After 2.5 h of reaction, the reaction kettle was cooled to room temperature. The reaction system was filtered, to separate out a solid part and a liquid part. The solid part was subjected to Soxhlet extraction with methanol to obtain 5.41 g of DMT, i.e., a monomer from polyester depolymerization. 3.48 g of undepolymerized polyester fibers were remained in the Soxhlet extractor. The liquid part was subjected to distillation to recover the methanol, ethylene glycol, and DBU (shown in Table 2).

**Comparative Example 5**

10 g of polyester fibers were washed with methanol at 50 °C for 30 min. 0.2 g of sodium carbonate was added during the washing, and the washed polyester fibers were dried at 75 °C for 1 h. The dried polyester fibers, 40 g of methanol, and 0.18 g of DBU were added into a reaction kettle and heated to 115 °C. After 2 h of reaction, the reaction kettle was cooled to room temperature. The reaction system was filtered, to separate out a solid part and a liquid part. The solid part was subjected to Soxhlet extraction with methanol to obtain 4.94 g of DMT, i.e., a monomer from polyester depolymerization. 3.25 g of undepolymerized polyester fibers were remained in the Soxhlet extractor. The liquid part was subjected to distillation to recover the methanol, ethylene glycol, and DBU (shown in Table 2).

**Comparative Example 6**

10 g of polyester fibers were washed with methanol at 50 °C for 30 min. 0.2 g of sodium carbonate was added during the washing, and the washed polyester fibers were dried at 75 °C for 1 h. The dried polyester fibers, 40 g of methanol, and 0.18 g of TBD were added into a reaction kettle and heated to 115 °C. After 2 h of reaction, the reaction kettle was cooled to room temperature. The reaction system was filtered, to separate out a solid part and a liquid part. The solid part was subjected to Soxhlet extraction with methanol to obtain 4.69 g of DMT, i.e., a monomer from polyester depolymerization. 3.61 g of undepolymerized polyester fibers were remained in the Soxhlet extractor. The liquid part was subjected to distillation to recover the methanol, ethylene glycol, and TBD (shown in Table 2).

Table 2 Comparisons between examples with the auxiliary catalyst and comparative examples without the auxiliary catalyst in terms of depolymerization efficiency and DMT monomer recovery rate

| Reaction temperature (°C) | Main catalyst | Auxiliary catalyst | Reaction example No. | Depolymerization efficiency E (%) | DMT recovery rate (mol%) |
|---|---|---|---|---|---|
| 65 | DBU | None | Comparative Example 1 | 54.50 | 27.85 |
| | | Acetonitrile | Example 1 | 100 | 88.62 |

(continued)

| Reaction temperature (°C) | Main catalyst | Auxiliary catalyst | Reaction example No. | Depolymerization efficiency E (%) | DMT recovery rate (mol%) |
|---|---|---|---|---|---|
| 85 | DBU | None | Comparative Example 2 | 49.30 | 39.55 |
| | | Acetonitrile | Example 2 | 100 | 92.68 |
| | | Benzonitrile | Example 3 | 100 | 85.94 |
| 90 | [HDBU][2-EtIm] | None | Comparative Example 3 | 62.50 | 45.10 |
| | | Acetonitrile | Example 4 | 100 | 86.24 |
| 95 | DBU | None | Comparative Example 4 | 65.20 | 53.62 |
| | | Acetonitrile | Example 5 | 100 | 95.16 |
| | | Adiponitrile | Example 6 | 100 | 84.75 |
| 115 | DBU | None | Comparative Example 5 | 67.50 | 48.97 |
| | | Acetonitrile | Example 7 | 100 | 85.31 |
| | | Propionitrile | Example 8 | 100 | 77.22 |
| 115 | TBD | None | Comparative Example 6 | 63.90 | 46.49 |
| | | Acetonitrile | Example 9 | 100 | 82.87 |

**Reference Example 10**

2 g of polyester fibers were washed with methanol at 60 °C for 30 min. 0.1 g of sodium carbonate was added during the washing, and the washed polyester fibers were dried at 75 °C for 1 h. The dried polyester fibers, 6.4 g of methanol, 1.6 g of acetonitrile, and 0.21 g of [HDBU][BIm] were added into a reaction kettle and heated to 95 °C. After 2 h of reaction, the reaction kettle was cooled to room temperature. The reaction system was filtered, to separate out a solid part and a liquid part. The solid part was subjected to Soxhlet extraction to obtain 1.82 g of DMT, i.e., a monomer from polyester depolymerization, without remaining solid matter in the Soxhlet extractor. The liquid part was subjected to distillation to recover the methanol and ethylene glycol, and [HDBU][BIm] was recovered through further extraction (shown in Table 3).

**Reference Examples 11 to 13**

The specific experimental steps and reaction conditions were the same as those in Example 7, except that the main catalyst was replaced in Examples 11 to 13, and the main catalysts in Examples 11 to 13 were [HDBU][2-MeIm], [HDBU][2-Et-4-MeIm], and [HDBU][2-PhIm], respectively. The experimental results are shown in Table 3.

Table 3 Polyester depolymerization rates and DMT monomer recovery rates in Examples 11 to 13

| Example No. | **10**([HDBU][BIm]) | **11**([HDBU][2-MeIm]) | 12([HDBU][2-Et-4-MeIm]) | 13([HDBU][2-PhIm]) |
|---|---|---|---|---|
| Depolymerization efficiency E(%) | 100 | 100 | 100 | 100 |
| DMT recovery rate (mol%) | 90.21 | 91.12 | 90.41 | 89.83 |

**Reference Example 14**

2 g of polyethylene-2,5-furandicarboxylate (PEF) fibers were washed with methanol at 60 °C for 30 min. 0.1 g of sodium carbonate was added during the washing, and the washed fibers were dried at 75 °C for 1 h. The dried fibers were added to a reaction kettle. 7.2 g of methanol, 0.8 g of acetonitrile, and 0.06 g of DBU were added to the reaction kettle. The reaction kettle was heated to 110 °C, and the reaction was performed for 2 h. After the reaction, the reaction kettle was cooled to room temperature. The reaction system was filtered, to separate out a solid part and a liquid part. The solid part was subjected to Soxhlet extraction with methanol to obtain 1.71 g of dimethyl furan-2,5-dicarboxylate (shown in FIG. 2), i.e., a monomer from polyester depolymerization. The liquid part was subjected to distillation to recover the methanol and acetonitrile, and ethylene glycol and DBU were recovered through further vacuum distillation.

**Reference Example 15**

10 g of polylactic acid (PLA) fibers were washed with methanol at 60 °C for 30 min. 0.2 g of sodium carbonate was added during the washing, and the washed fibers were dried at 75 °C for 1 h. The dried fibers were added to a reaction kettle. 36 g of methanol, 4 g of acetonitrile, and 0.3 g of DBU were added to the reaction kettle. The reaction kettle was heated to 100 °C, and the reaction was performed for 2 h. After the reaction, the reaction kettle was cooled to room temperature. The PLA fibers were completely depolymerized. The reaction solution was subjected to distillation to recover the methanol and acetonitrile, and 11.31 g of methyl lactate was recovered through further vacuum distillation.

**Example 16 -According to the present invention-**

2 g of polyester fibers and 2 g of cotton fibers were washed with methanol at 60 °C for 30 min. 0.1 g of sodium carbonate was added during the washing, and the washed fibers were dried at 75 °C for 1 h. The dried fibers were added to a reaction kettle. 9.2 g of methanol, 0.8 g of acetonitrile, and 0.45 g of DBU were added to the reaction kettle. The reaction kettle was heated to different temperatures (ranging from 100 °C to 115 °C), and reactions at different temperatures were performed for 2 h separately. After the reaction, the reaction kettle was cooled to room temperature. The reaction system was filtered, to separate out a solid part and a liquid part. The polyester fibers were completely depolymerized. The solid part was subjected to Soxhlet extraction with methanol to obtain cotton fibers. The cotton fibers had recovery rates of 98% (reaction temperature being at 100 °C) and 94% (reaction temperature being 115 °C), respectively. The physical properties of the recovered cotton fibers are shown in Table 4.

**Comparative Example 7**

2 g of polyester fibers and 2 g of cotton fibers were washed with methanol at 60 °C for 30 min. 0.1 g of sodium carbonate was added during the washing, and the washed fibers were dried at 75 °C for 1 h. The dried fibers were added to a reaction kettle. 10 of methanol and 0.45 g of DBU were added to the reaction kettle. The reaction kettle was heated to different temperatures (ranging from 190 °C to 240 °C), reactions at different temperatures were performed for 2 h separately. After the reaction, the reaction kettle was cooled to room temperature. The reaction system was filtered, to separate out a solid part and a liquid part. The polyester fibers were completely depolymerized. The solid part was subjected to Soxhlet extraction with methanol to separate out cotton fibers and DMT, i.e., a monomer from polyester depolymerization. The liquid part was subjected to distillation to recover the methanol and acetonitrile, and ethylene glycol and DBU were recovered through further vacuum distillation. The physical properties of the recovered cotton fibers are shown in Table 4.

Table 4 Physical properties of recovered cotton fibers in examples and comparative examples at different reaction temperatures

| Cotton fibers | | Titer, dtex | Cotton toughness, cN/dtex | Fiber elongation, % |
|---|---|---|---|---|
| | New cotton fibers | 1.38±0.41 | 4.26±0.87 | 8.26±1.37 |
| Example 16 | Cotton fibers recovered at 100 °C | 1.40±0.35 | 4.05±0.96 | 7.35±1.33 |
| | Cotton fibers recovered at 115 °C | 1.76±0.42 | 3.85±1.12 | 8.53±1.65 |
| Comparative Example 7 | Cotton fibers recovered at 190 °C | 1.83±0.31 | 2.3±0.78 | 5.31±1.67 |
| | Cotton fibers recovered at 240 °C | The cotton fibers became powdery and physical properties thereof could not be measured | | |

**Example 17 -According to the present invention-**

200 g of polyester-cotton blend fabric (cotton content of 50%) was washed with methanol at 100 °C for 45 min. 4 g of $Na_2CO_3$ was added during the washing, and the washed fabric was dried at 75 °C for 1 h. The dried blend fabric was added to a reaction kettle. 752 g of methanol, 48 g of acetonitrile, and 5 g of DBU were simultaneously added to the reaction kettle. The reaction kettle was heated to 110 °C, and the reaction was performed for 2 h. After the reaction, the reaction kettle was cooled to room temperature. The reaction system was filtered, to separate out a solid part and a liquid part. The

solid part was subjected to Soxhlet extraction with methanol to obtain 98.6 g of a cotton fabric component and 184.2 g of DMT, i.e., a monomer from polyester depolymerization. The liquid part was subjected to distillation to recover the methanol and acetonitrile, and ethylene glycol and DBU were recovered through further vacuum distillation. The cotton fabric component was analyzed by gas chromatography according to GB/T2912.3-2009 and GB/T7717.12-94, and no acetonitrile was remained in the cotton fibers. The recovered cotton fabric component (shown in FIG. 3) was opened by an opening machine to obtain cotton fibers. These recovered cotton fibers were blend-spun with new cotton fibers to produce regenerated yarns. The physical properties of some regenerated cotton yarns were shown in Table 5.

Table 5 Mechanical properties of yarns regenerated from recovered cotton fabric component after reaction and separation of polyester component

| Cotton yarn composition | Titer, tex | Strength, cN/tex |
|---|---|---|
| 100% new cotton | 60 | 8.86 |
| 30% recovered cotton/70% new cotton | 60 | 9.52 |
| 50% recovered cotton/50% new cotton | 58 | 9.27 |
| 70% recovered cotton/30% new cotton | 62 | 8.53 |

**Example 18 -According to the present invention-**

8 g of polyester-polyurethane blended printed fabric (polyurethane content of 15%) was washed with methanol at 85 °C for 30 min. 0.08 g of sodium acetate and 0.08 g of calcium oxide were added during the washing, and the washed fabric was vacuum dried at 45 °C for 1 h. The dried blend fabric (shown in A of FIG. 4) was added to a reaction kettle. 28.8 g of methanol, 3.2 g of acetonitrile, and 0.4 g of DBU were simultaneously added to the reaction kettle. The reaction kettle was heated to 90 °C, and the reaction was performed for 2 h. After the reaction, the reaction kettle was cooled to room temperature. The reaction system was filtered, to separate out a solid part and a liquid part. The solid part was subjected to Soxhlet extraction to obtain 1.16 g of a polyurethane component (shown in B of FIG. 4 and C of FIG. 4) and 6.40 g of DMT, i.e., a monomer from polyester depolymerization. The liquid part was subjected to distillation to recover the methanol and acetonitrile, and ethylene glycol and DBU were recovered through further vacuum distillation.

FIG. 4 shows that the polyurethane recovered from the printed blend fabric is pure white, and polyurethane filaments are not damaged and could be directly used as a raw material to produce regenerated polyurethane filaments.

**Comparative Example 8**

8 g of polyester-polyurethane blended printed fabric (polyurethane content of 15%) was washed with methanol at 85 °C for 30 min. 0.08 g of sodium acetate and 0.08 g of calcium oxide were added during the washing, and the washed fabric was vacuum dried at 45 °C for 1 h. The dried blend fabric (shown in A of FIG. 4) was added to a reaction kettle. 32 of methanol and 0.4 g of DBU were simultaneously added to the reaction kettle. The reaction kettle was heated to 125 °C, and the reaction was performed for 2 h. After the reaction, the reaction kettle was cooled to room temperature. The reaction system was filtered, to separate out a solid part and a liquid part. The solid part was subjected to Soxhlet extraction to obtain only 6.67 g of DMT, i.e., a monomer from polyester depolymerization, without the polyurethane component. The polyurethane had been completely degraded at this reaction temperature and could not be recycled.

**Example 19 -According to the present invention-**

8 g of polyester-polyurethane blended dyed fabric (black, polyurethane content of 15%) was washed with methanol at 85 °C for 30 min. 0.08 g of sodium acetate and 0.08 g of calcium oxide were added during the washing, and the washed fabric was vacuum dried at 45 °C for 1 h. The dried blend fabric was added to a reaction kettle. 28.8 g of methanol, 3.2 g of acetonitrile, and 0.4 g of DBU were simultaneously added to the reaction kettle. The reaction kettle was heated to 90 °C, and the reaction was performed for 2 h. After the reaction, the reaction kettle was cooled to room temperature. The reaction system was filtered, to separate out a solid part and a liquid part. The solid part was subjected to Soxhlet extraction to obtain 1.16 g of a polyurethane component and 6.36 g of DMT, i.e., a monomer from polyester depolymerization. The liquid part was subjected to distillation to recover the methanol and acetonitrile, and ethylene glycol and DBU were recovered through further vacuum distillation. The polyurethane component recovered from the black printed blend fabric was light brown. The polyurethane filaments were not damaged and could be directly used as a raw material to produce regenerated polyurethane filaments.

**Example 20 -According to the present invention-**

8 g of polyester-wool blend fabric (wool content of 65%) was washed with methanol at 85 °C for 30 min. 0.08 g of sodium acetate and 0.08 g of calcium oxide were added during the washing, and the washed fabric was vacuum dried at 65 °C for 1 h. The dried blend fabric was added to a reaction kettle. 30 g of methanol, 2 g of acetonitrile, and 0.61 g of DBU were simultaneously added to the reaction kettle. The reaction kettle was heated to 95 °C, and the reaction was performed for 2 h. After the reaction, the reaction kettle was cooled to room temperature. The reaction system was filtered, to separate out a solid part and a liquid part. The solid part was subjected to Soxhlet extraction to obtain 5.1 g of a wool component and 2.64 g of DMT, i.e., a monomer from polyester depolymerization. The liquid part was subjected to distillation to recover the methanol and acetonitrile, and ethylene glycol and DBU were recovered through further vacuum distillation. The separated wool could be recycled to produce regenerated wool.

**Example 21 -According to the present invention-**

5 g of nylon fabric and 5 g of polyester fabric were washed with methanol at 95 °C for 45 min. 0.08 g of sodium carbonate and 0.08 g of calcium oxide were added during the washing, and the washed mixed fabrics were vacuum dried at 95 °C for 1 h. The dried mixed fabrics were added to a reaction kettle. 36 g of methanol, 4 g of acetonitrile, and 0.3 g of DBU were simultaneously added to the reaction kettle. The reaction kettle was heated to 100 °C, and the reaction was performed for 2 h. After the reaction, the reaction kettle was cooled to room temperature. The reaction system was filtered, to separate out a solid part and a liquid part. The solid part was subjected to Soxhlet extraction to obtain 4.91 g of a nylon component and 4.71 g of DMT, i.e., a monomer from polyester depolymerization. The liquid part was subjected to distillation to recover the methanol and acetonitrile, and ethylene glycol and DBU were recovered through further vacuum distillation. The separated nylon was 100% pure nylon according to infrared spectrum analysis, and could be recycled to produce regenerated nylon filaments.

**Example 22 -According to the present invention-**

100 g of polyester fibers were washed with methanol at 50 °C for 30 min. 0.2 g of sodium carbonate was added during the washing, and the washed polyester fibers were dried at 75 °C for 1 h. The dried polyester fibers, 360 g of methanol, 40 of acetonitrile, and 3 g of DBU were added into a reaction kettle and the reaction kettle was then heated to 95 °C. After 2 h of reaction, the reaction kettle was cooled to room temperature. The reaction system was filtered, to separate out a solid part and a liquid part. The solid part was subjected to Soxhlet extraction with methanol to obtain DMT, i.e., a monomer from polyester depolymerization. The obtained monomer from polyester was polymerized under vacuum at 290 °C for 3 h in the presence of antimony trioxide as a catalyst to obtain a polyester. The obtained polyester was subjected to repeated depolymerization and polymerization multiple times, with parameters of each depolymerization and condensation polymerization shown in Table 6.

Table 6 Parameters for repeated polymerization and depolymerization of the monomer(s) from polyester depolymerization

| | First depolymerization and polymerization | Second depolymerization and polymerization | Third depolymerization and polymerization |
|---|---|---|---|
| Polyester depolymerization rate, % | 100 | 100 | 100 |
| DMT recovery rate, mol% | 93.18 | 94.02 | 92.76 |
| Yield of polyester polymerization, mol% | 86.52 | 88.17 | 87.63 |
| Intrinsic viscosity of polyester, dL/g | 0.63 | 0.65 | 0.62 |
| Recycling rate of recovered polyester, % | 80.62 | 66.83 | 54.32 |

**Claims**

**1.** A method for recycling waste materials made by blend-spinning temperature-sensitive fibers with polyester, comprising the steps of

treating a polyester-containing raw material to be treated by

washing the polyester-containing raw material to be treated with an alcohol or an alcohol-containing mixed system at a temperature of 40 °C to 130 °C for 0.3 h to 2 h, wherein
the alcohol-containing mixed system is a mixture of an alcohol with an alkaline compound, and the alkaline compound is at least one selected from the group consisting of sodium carbonate, sodium acetate, sodium methoxide, sodium ethoxide, potassium carbonate, potassium acetate, potassium methoxide, calcium oxide, and calcium hydroxide;
adding a resulting treated polyester-containing raw material into a methanol solution, and subjecting a resulting mixture to polyester degradation at a temperature of 60 °C to 110 °C for 1 h to 5 h in the presence of a catalyst, to achieve 100% depolymerization, wherein
the catalyst consists of an organic alkali main catalyst and an auxiliary catalyst, and the catalyst is in an amount of 0.1 wt% to 20 wt% of a mass of the polyester-containing raw material to be treated; a mass ratio of the organic alkali main catalyst to the auxiliary catalyst ranges from 1 : 0.01 to 1 : 100; the organic alkali main catalyst is at least one selected from the group consisting of a nitrogen-containing amidine compound, and a nitrogen-containing guanidine compound; and the auxiliary catalyst is a nitrile compound;
separating out and recovering a polyester component by:

conducting solid-liquid separation after the polyester degradation, collecting a resulting solid, and subjecting the resulting solid to Soxhlet extraction to separate the polyester component from other temperature-sensitive fiber components;
subjecting a resulting liquid to atmospheric distillation to recover methanol and the auxiliary catalyst; and
subjecting a resulting residue after the atmospheric distillation to vacuum distillation or extraction to recover a diol and a binary acid that are monomers from polyester, as well as the organic alkali main catalyst; and
reutilizing the polyester component and other temperature-sensitive fiber components.

**2.** The method for recycling the polyester blend fabric as claimed in claim 1, wherein the organic alkali main catalyst is one selected from the group consisting of

a) 1,8-diazabicyclo[5.4.0]undec-7-ene (DBU), a DBU-supported polymer or compound, an organic salt formed from DBU and imidazole, and an organic salt formed from DBU and a derivative of imidazole;
b) 1,5,7-triazabicyclo[4.4.0]dec-5-ene (TBD), a TBD-supported polymer or compound, an organic salt formed from TBD and imidazole, and an organic salt formed from TBD and a derivative of imidazole; and
c) 1,5-diazabicyclo[4.3.0]non-5-ene (DBN), a DBN-supported polymer or compound, an organic salt formed from DBN and imidazole, and an organic salt formed from DBN and a derivative of imidazole.

**3.** The method for recycling the polyester blend fabric as claimed in claim 1, wherein the auxiliary catalyst is at least one selected from the group consisting of acetonitrile, propionitrile, benzonitrile, and adiponitrile.

**4.** The method for recycling the polyester blend fabric as claimed in claim 1, wherein the polyester-containing raw material to be treated is a mixed fabric of polyester fabric with other fabrics;

the mixed fabric includes 5% **to** 100% of a polyester; and
the polyester blend fabric is a fabric prepared by blend-spinning polyester with at least one selected from the group consisting of polyurethane, cotton, viscose fiber, a regenerated cellulose fiber, nylon, wool, cashmere, and silk.

## Patentansprüche

**1.** Verfahren zum Recycling von Abfallmaterialien, die durch Mischspinnen temperaturempfindlicher Fasern mit Polyester hergestellt wurden, umfassend die Schritte

Behandeln eines zu behandelnden polyesterhaltigen Rohmaterials durch
Waschen des zu behandelnden polyesterhaltigen Rohmaterials mit einem Alkohol oder einem alkoholhaltigen Mischsystem bei einer Temperatur von 40 °C bis 130 °C für 0,3 h bis 2 h, wobei
das alkoholhaltige Mischsystem eine Mischung aus einem Alkohol und einer alkalischen Verbindung ist und die alkalische Verbindung mindestens eine Verbindung ist, die aus der Gruppe ausgewählt ist, bestehend aus Natriumcarbonat, Natriumacetat, Natriummethoxid, Natriumethoxid, Kaliumcarbonat, Kaliumacetat, Kaliummethoxid, Calciumoxid und Calciumhydroxid;

Hinzufügen eines resultierenden behandelten polyesterhaltigen Rohmaterials zu einer Methanollösung und Unterziehen einer resultierenden Mischung einem Polyesterabbau bei einer Temperatur von 60 °C bis 110 °C für 1 Stunde bis 5 Stunden in Gegenwart eines Katalysators, um eine 100%ige Depolymerisation zu erreichen, wobei

der Katalysator aus einem organischen Alkali-Hauptkatalysator und einem Hilfskatalysator besteht und der Katalysator in einer Menge von 0,1 Gew.-% bis 20 Gew.-% der Masse des zu behandelnden polyesterhaltigen Rohmaterials vorliegt; das Massenverhältnis des organischen Alkali-Hauptkatalysators zum Hilfskatalysator im Bereich von 1 : 0,01 bis 1 : 100 liegt; der organische Alkali-Hauptkatalysator mindestens eine Verbindung ist, ausgewählt aus der Gruppe bestehend aus einer stickstoffhaltigen Amidinverbindung und einer stickstoffhaltigen Guanidinverbindung; und der Hilfskatalysator eine Nitrilverbindung ist;

Abtrennung und Rückgewinnung einer Polyesterkomponente durch:

Durchführung einer Fest-Flüssig-Trennung nach dem Polyesterabbau, Sammeln eines resultierenden Feststoffs und Unterziehen des resultierenden Feststoffs einer Soxhlet-Extraktion, um die Polyesterkomponente von anderen temperaturempfindlichen Faserkomponenten abzutrennen;

Unterziehen der resultierenden Flüssigkeit einer atmosphärischen Destillation, um Methanol und den Hilfskatalysator zurückzugewinnen; und

Unterziehen eines nach der atmosphärischen Destillation resultierenden Rückstands einer Vakuumdestillation oder -extraktion, um ein Diol und eine binäre Säure, die Monomere des Polyesters sind, sowie den organischen Alkali-Hauptkatalysator zurückzugewinnen; und Wiederverwendung der Polyesterkomponente und anderer temperaturempfindlicher Faserkomponenten.

**2.** Verfahren zum Recycling des Polyestermischgewebes nach Anspruch 1, wobei der organische Alkali-Hauptkatalysator einer ist, der aus der Gruppe ausgewählt ist, bestehend aus

a) 1,8-Diazabicyclo[5.4.0]undec-7-en (DBU), ein DBU-getragenes Polymer oder eine DBUgetragene Verbindung, ein aus DBU und Imidazol gebildetes organisches Salz und ein aus DBU und einem Imidazolderivat gebildetes organisches Salz;

b) 1,5,7-Triazabicyclo[4.4.0]dec-5-en (TBD), ein TBD-getragenes Polymer oder eine TBDgetragene Verbindung, ein aus TBD und Imidazol gebildetes organisches Salz und ein aus TBD und einem Imidazolderivat gebildetes organisches Salz; und

c) 1,5-Diazabicyclo[4.3.0]non-5-en (DBN), ein DBN-getragenes Polymer oder eine DBNgetragene Verbindung, ein aus DBN und Imidazol gebildetes organisches Salz und ein aus DBN und einem Imidazolderivat gebildetes organisches Salz.

**3.** Verfahren zum Recycling des Polyestermischgewebes nach Anspruch 1, wobei der Hilfskatalysator mindestens eine Verbindung ist, die aus der Gruppe ausgewählt ist, bestehend aus Acetonitril, Propionitril, Benzonitril und Adiponitril.

**4.** Verfahren zum Recycling des Polyestermischgewebes nach Anspruch 1, wobei das zu behandelnde polyesterhaltige Rohmaterial ein Mischgewebe aus Polyestergewebe und anderen Geweben ist;

das Mischgewebe 5 % bis 100 % eines Polyesters enthält; und

das Polyestermischgewebe ein Gewebe ist, das durch Mischspinnen von Polyester mit mindestens einem aus der Gruppe bestehend aus Polyurethan, Baumwolle, Viskosefaser, einer regenerierten Zellulosefaser, Nylon, Wolle, Kaschmir und Seide hergestellt wurde.

## Revendications

**1.** Procédé de recyclage de déchets obtenus par filage par mélange de fibres thermosensibles avec du polyester, comprenant les étapes comprenant :

traiter une matière première contenant du polyester à traiter en

lavant ladite matière première contenant du polyester à traiter avec un alcool ou un système mixte contenant de l'alcool à une température comprise entre 40 °C et 130 °C pendant 0,3 h à 2 h, dans lequel

le système mixte contenant de l'alcool est un mélange d'un alcool et d'un composé alcalin, et le composé alcalin est au moins un composé choisi dans le groupe constitué par le carbonate de sodium, l'acétate de sodium, le méthylate de sodium, l'éthylate de sodium, le carbonate de potassium, l'acétate de potassium, le méthylate de

potassium, l'oxyde de calcium et l'hydroxyde de calcium ;
ajouter une matière première contenant du polyester traitée ainsi obtenue dans une solution de méthanol, et soumettre le mélange résultant à une dégradation du polyester à une température comprise entre 60 °C et 110 °C pendant 1 h à 5 h en présence d'un catalyseur, afin d'obtenir une dépolymérisation à 100 %, dans lequel le catalyseur est constitué d'un catalyseur principal alcalin organique et d'un catalyseur auxiliaire, et le catalyseur est présent en une quantité de 0,1 % en poids à 20 % en poids par rapport à la masse de la matière première contenant du polyester à traiter ; le rapport massique entre le catalyseur principal alcalin organique et le catalyseur auxiliaire est compris entre 1 : 0,01 et 1 : 100 ; le catalyseur principal alcalin organique est au moins un composé choisi dans le groupe constitué d'un composé d'amidine azoté et d'un composé de guanidine azoté ; et le catalyseur auxiliaire est un composé nitrile ;
séparer et récupérer un composant polyester en :

effectuant une séparation solide-liquide après la dégradation du polyester, en recueillant un solide résultant, et en soumettant le solide résultant à une extraction de Soxhlet pour séparer le composant polyester des autres composants fibreux thermosensibles ;
soumettre le liquide obtenu à une distillation atmosphérique pour récupérer le méthanol et le catalyseur auxiliaire ; et
soumettre le résidu obtenu après la distillation atmosphérique à une distillation sous vide ou à une extraction pour récupérer un diol et un acide binaire qui sont des monomères du polyester, ainsi que le catalyseur principal alcalin organique ; et réutiliser le composant polyester et les autres composants fibreux thermosensibles.

**2.** Procédé de recyclage du tissu en mélange de polyester selon la revendication 1, dans lequel le catalyseur principal alcalin organique est choisi dans le groupe constitué de

a) le 1,8-diazabicyclo[5.4.0]undéc-7-ène (DBU), un polymère ou un composé supporté par le DBU, un sel organique formé à partir du DBU et de l'imidazole, et un sel organique formé à partir du DBU et d'un dérivé de l'imidazole ;
b) le 1,5,7-triazabicyclo[4.4.0]déc-5-ène (TBD), un polymère ou un composé supporté par le TBD, un sel organique formé à partir du TBD et de l'imidazole, et un sel organique formé à partir du TBD et d'un dérivé de l'imidazole ; et
c) le 1,5-diazabicyclo[4.3.0]non-5-ène (DBN), un polymère ou un composé supporté par le DBN, un sel organique formé à partir du DBN et de l'imidazole, et un sel organique formé à partir du DBN et d'un dérivé de l'imidazole.

**3.** Procédé de recyclage du tissu en mélange de polyester selon la revendication 1, dans lequel le catalyseur auxiliaire est au moins un élément choisi dans le groupe constitué par l'acétonitrile, le propionitrile, le benzonitrile et l'adiponitrile.

**4.** Procédé de recyclage du tissu en mélange de polyester selon la revendication 1, dans lequel la matière première contenant du polyester à traiter est un tissu mélangé composé de tissu en polyester et d'autres tissus ;

le tissu mélangé comprend 5 % à 100 % de polyester ; et
le tissu en mélange de polyester est un tissu préparé par filage de mélange de polyester avec au moins un élément choisi parmi le groupe constitué du polyuréthane, du coton, de la fibre de viscose, d'une fibre de cellulose régénérée, du nylon, de la laine, du cachemire et de la soie.

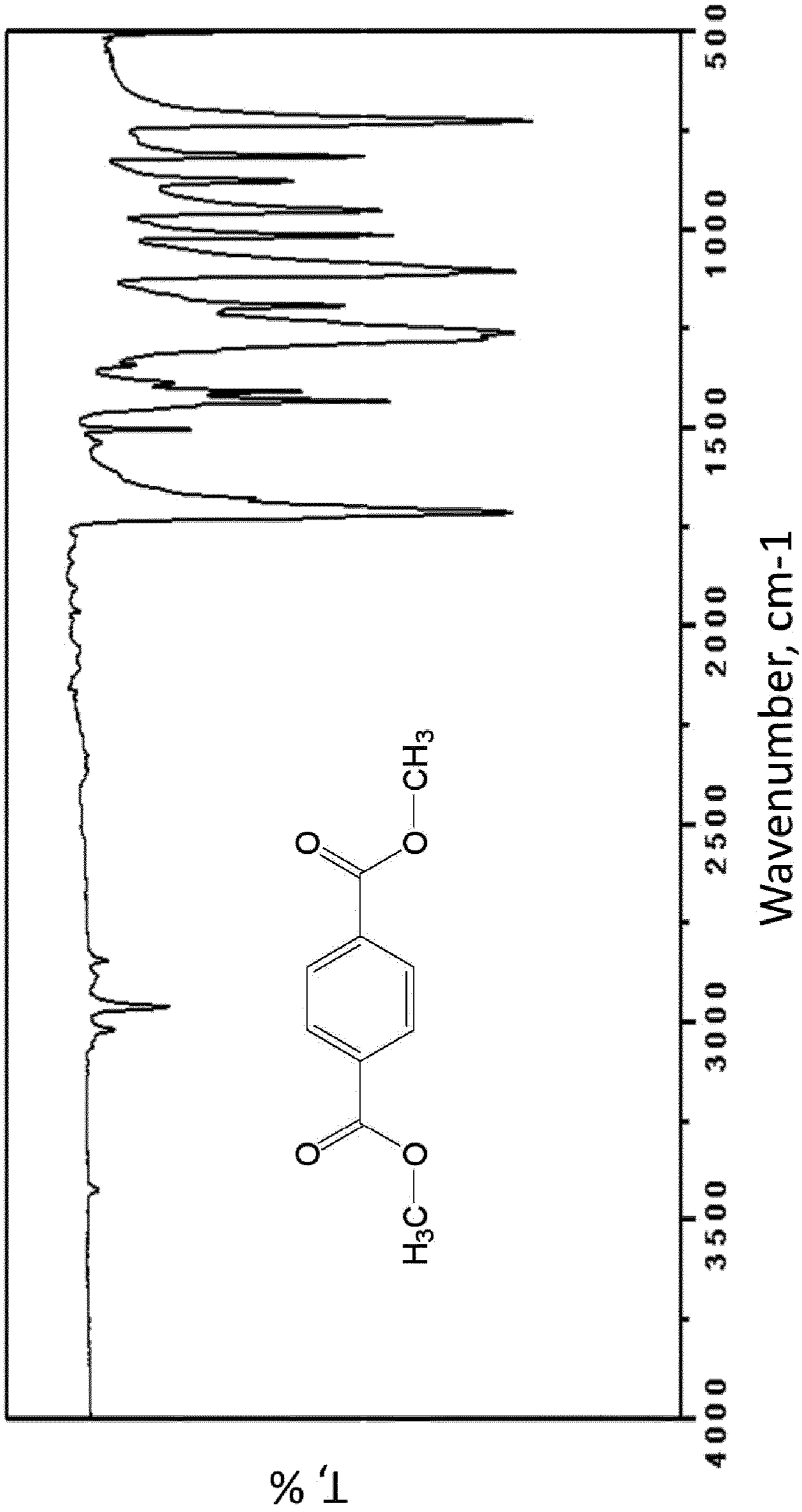


FIG. 1

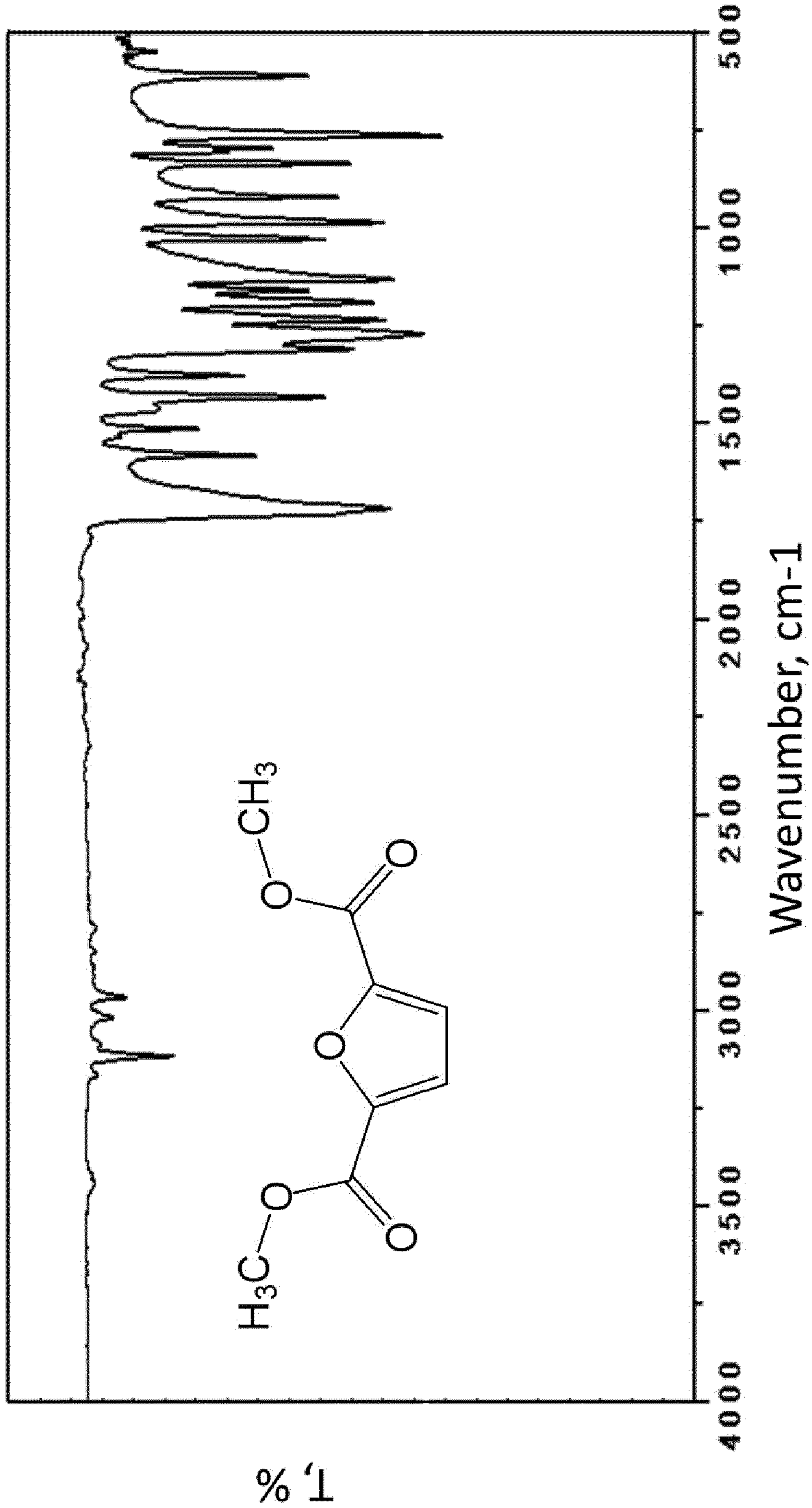


FIG. 2

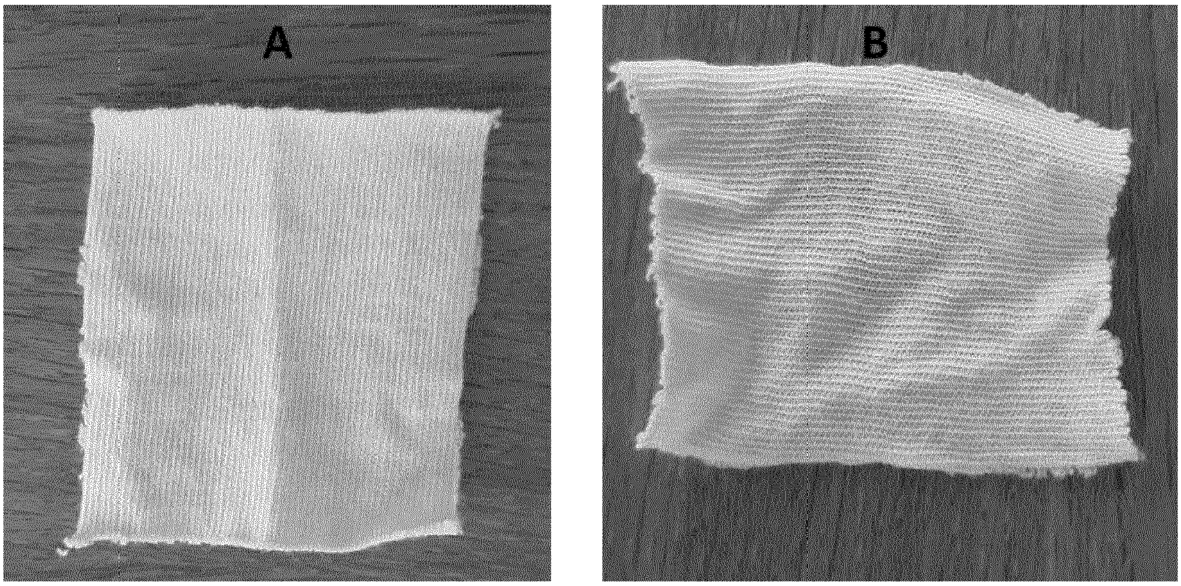


FIG. 3

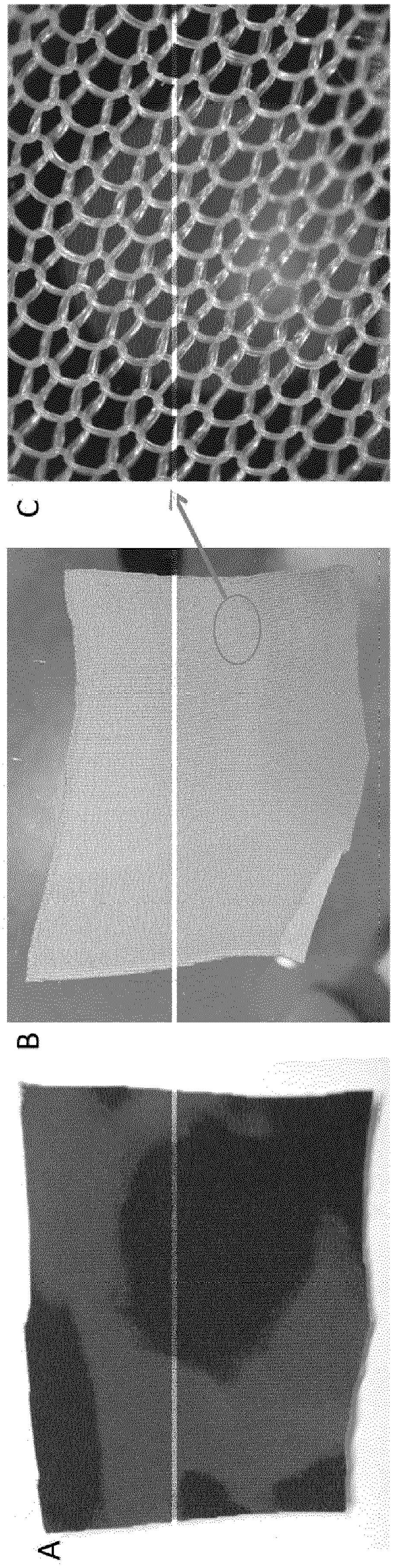


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 106113319 A
- CN 102250379 B
- US 8541477 B2
- WO 2021126661 A1
- WO 2019140245 A1
- WO 2020035590 A1
- US 9255194 B2


### Non-patent literature cited in the description


- **PALME, A. et al.** Development of an efficient route for combined recycling pf PET and cotton from mixed fabrics. *Text Cloth Sustain*, 2017, vol. 3, 4
- *GB/T2912.3-2009*
- *GB/T7717.12-94*